# EUROPEAN PATENT SPECIFICATION

(11) **EP 3 524 147 B1**
(45) Date of publication and mention of the grant of the patent: **13.09.2023**
(21) Application number: 18156204.2
(22) Date of filing: 12.02.2018
(51) Int. Cl.: A61B 5/053

(54) **BODY IMPEDANCE MEASUREMENT APPARATUS AND METHOD**
KÖRPERIMPEDANZMESSVORRICHTUNG UND -VERFAHREN
APPAREIL DE MESURE D'IMPÉDANCE CORPORELLE ET PROCÉDÉ

(43) Date of publication of application: 14.08.2019
(73) Proprietor: Nokia Technologies Oy, 02610 Espoo (FI)
(72) Inventor: MATIC, Tomislav, 31000 Osijek (HR); HERCEG, Marijan, 31000 Osijek (HR); JOB, Josip, 31000 Osijek (HR); GRBIC, Ratko, 31000 Osijek (HR)
(74) Representative: Sayer, Robert David

(56) References cited:
- CN-B- 102 579 033
- US-A1- 2006 085 049
- US-A1- 2012 172 742
- US-A1- 2015 051 505

## Description

### TECHNICAL FIELD

An example embodiment relates generally to a body impedance measurement apparatus and method and, more particularly, to a body impedance measurement apparatus and method in which the transmitting electrode and the receiving electrode wirelessly communicate via the body.

### BACKGROUND

A variety of different monitoring devices have been developed to be worn by a subject and to measure various parameters. One such monitor is a bio-impedance monitor that monitors the impedance of a portion of the body. The impedance provides information regarding the composition of the portion of the body being interrogated, that is, the types of tissue, such as fat, muscle or the like, that form the portion of the body that is being interrogated. As a result, body impedance may be monitored for a variety of purposes including the collection of information indicative of the overall health of the subject as well as information for use in conjunction with rehabilitation and training of the subject in which changes in the body impedance over time are monitored in order to track the rehabilitation or training regimen of the subject.

Bio-impedance monitors generally inject an alternating current (AC) between the pair of transmitting electrodes and monitor the resulting voltage between a pair of receiving electrodes. The pair of transmitting electrodes and the pair of receiving electrodes are each generally wirelessly connected to facilitate the provision of the AC current and the measurement of the resulting voltage, respectively.

US2006/085049 discloses an active electrode tissue discrimination system.

### BRIEF SUMMARY

The invention is set forth in the claims. A body impedance measurement apparatus and method as well as the transmitter and receiver of a body impedance measurement apparatus are provided in accordance with certain example embodiments. The body impedance measurement apparatus interacts with a transmitting electrode and a receiving electrode that are in wireless communication via the body. The body impedance measurement apparatus and method therefore permit information from which a measure of the body impedance may be determined while relying upon wireless communication between the transmitting and receiving electrodes. As such, the body impedance measurement apparatus and method may be more readily integrated into a body worn system for monitoring the body impedance without inhibiting the subject as the subject performs their daily activities. Additionally, the body impedance measurement apparatus and method of an example embodiment are configured to communicate with a user interface, such as the user interface of another device worn or carried by the subject, such as a smartwatch, a smartphone or the like, so as to present information regarding the body impedance, thereby facilitating provision of information regarding the body impedance to the subject wearing the body impedance measurement apparatus.

In an example embodiment, an apparatus is provided that includes transmitter means for providing an amplitude modulated signal via a transmitting electrode means to a body to which the transmitting electrode means is configured to be applied. The body impedance measurement apparatus also includes receiver means for receiving signals, responsive to the amplitude modulated signal, via a receiving electrode means that is configured to be applied to the body and to be remote from the transmitting electrode means so as to be a wireless communication with the transmitting electrode means via the body. The receiver means is configured to process the received signals to determine a measure of the body impedance.

The transmitter means also includes modulator means configured to modulate an amplitude of a first signal in accordance with an amplitude modulation waveform to generate the amplitude modulated signal for which the amplitude modulation is repeated at a predefined frequency. In an example embodiment in which the amplitude modulation waveform comprises a sawtooth envelope, the modulator means is configured to modulate the amplitude of the first signal to generate the amplitude modulated signal having a sawtooth shape that repeats at the predefined frequency. The receiver means of an example embodiment also includes comparator means configured to compare the received signals to a threshold level. In this example embodiment, the comparator means may also be configured to generate a rectangular output waveform having the predefined frequency. The rectangular output waveform also has a duty cycle that is indicative of the measure of the body impedance. The receiver means of an example embodiment also includes processor means configured to determine the measure of the body impedance based upon the duty cycle.

In another example embodiment, a body impedance measurement apparatus is provided that includes a transmitter configured to provide an amplitude modulated signal via a transmitting electrode to a body to which the transmitting electrode is configured to be applied. The body impedance measurement apparatus also includes a receiver for receiving signals, responsive to the amplitude modulated signal, via a receiving electrode that is configured to be applied to the body and to be remote from the transmitting electrode so as to be in wireless communication with the transmitting electrode via the body. The receiver is also configured to process the received signals to determine a measure of the body impedance.

The transmitter of an example embodiment also includes a modulator configured to modulate an amplitude of a first signal in accordance with an amplitude modulation waveform to generate the amplitude modulated signal for which the amplitude modulation is repeated at a predefined frequency. In an example embodiment in which the amplitude modulation waveform comprises a sawtooth envelope, the modulator is configured to modulate the amplitude of the first signal in accordance with the sawtooth envelope to generate the amplitude modulated signal having a sawtooth shape that repeats at the predefined frequency. The receiver of an example embodiment also includes a comparator configured to compare the received signals to a threshold level. In this example embodiment, the receiver may also be configured to generate a rectangular output waveform having the predefined frequency. The rectangular output waveform also has a duty cycle that is indicative of the measure of the body impedance. The receiver of an example embodiment also includes a processor configured to determine the measure of the body impedance based upon the duty cycle.

In an example embodiment, a transmitter of the body impedance measurement apparatus is provided. The transmitter includes signal generator means configured to generate a first signal. The transmitter also includes modulator means configured to modulate an amplitude of the first signal in accordance with an amplitude modulation waveform to generate an amplitude modulated signal for which the amplitude modulation is repeated at a predefined frequency. In an example embodiment in which the amplitude modulation waveform comprises a sawtooth envelope, the modulator means is configured to modulate the amplitude of the first signal in accordance with the sawtooth envelope to generate the amplitude modulated signal having a sawtooth shape that repeats at the predefined frequency. The amplitude modulated signal is provided to transmitting electrode means that is configured to be applied to a body and that is also configured to transmit the amplitude modulated signal through the body to a remote receiver in wireless communication with the transmitter via the body.

In another example embodiment, a transmitter of a body impedance measurement apparatus is provided. The transmitter includes a signal generator configured to generate a first signal and a modulator configured to modulate an amplitude of the first signal in accordance with an amplitude modulated waveform to generate an amplitude modulated signal for which the amplitude modulation is repeated at a predefined frequency. In an embodiment in which the amplitude modulation waveform comprises a sawtooth envelope, the modulator is configured to modulate the amplitude of the first signal in accordance with the sawtooth envelope to generate the amplitude modulated signal having a sawtooth shape that repeats at the predefined frequency. The amplitude modulated signal is provided to a transmitting electrode that is configured to be applied to a body and that is also configured to transmit the amplitude modulated signal through the body to a remote receiver in wireless communication with the transmitter via the body.

In an example embodiment, a receiver of the body impedance measurement apparatus is provided. The receiver includes comparator means configured to be responsive to the received signals received via a receiving electrode means that is applied to a body so as to be in wireless communication with a remote transmitter via the body in order to receive signal following propagation through the body. The comparator means is configured to compare the received signals to a threshold level and to generate a rectangular output waveform having a duty cycle. The receiver further includes processor means, responsive to the comparator means, configured to determine a measure of the body impedance based upon the duty cycle. In an example embodiment in which the received signals are responsive to an amplitude modulated signal for which amplitude modulation is repeated at a predefined frequency, the rectangular output waveform generated by the comparator means also has the predefined frequency.

In another example embodiment, a receiver of the body impedance measurement apparatus is provided. The receiver includes a comparator configured to be responsive to received signals received via a receiving electrode that is applied to a body so as to be in wireless communication with a remote transmitter via the body in order to receive signals following propagation through the body. The comparator is also configured to compare the received signals to a threshold level and to generate a rectangular output waveform having a duty cycle. The receiver further includes a processor, responsive to the comparator, configured to determine a measure of the body impedance based upon the duty cycle. In an example embodiment in which the received signals are responsive to an amplitude modulated signal for which amplitude modulation is repeated at a predefined frequency, the rectangular output waveform generated by the comparator also has the predefined frequency.

In an example embodiment, a method for measuring body impedance is provided that includes causing an amplitude modulated signal to be provided to a transmitting electrode that is applied to a body such that the amplitude modulated signal is transmitted through the body. The method also includes receiving signals, responsive to the amplitude modulated signal, via a receiving electrode that is applied to the body and is remote from the transmitting electrode so as to be in wireless communication with the transmitting electrode via the body. The method further includes processing the received signals to determine a measure of the body impedance.

The method of an example embodiment also includes modulating the amplitude of a first signal in accordance with an amplitude modulation waveform to generate the amplitude modulated signal for which the amplitude modulation is repeated at a predefined frequency. In an embodiment in which the amplitude modulation waveform comprises a sawtooth envelope, the modulation of the amplitude of the first signal may include modulation of the amplitude of the first signal in accordance with the sawtooth envelope to generate the amplitude modulated signal having a sawtooth shape that repeats at the predefined frequency. The method of an example embodiment also includes analog processing of the received signals from the receiving electrode to remove negative signals prior to processing the received signals. The method of an example embodiment also includes comparing the received signals to a threshold level prior to processing the received signals. The method of an example embodiment also includes comparing the received signals to a threshold level to generate a rectangular output waveform having the predefined frequency and a duty cycle. In this example embodiment, the processing of the received signals includes determining the measure of the body impedance based upon the duty cycle.

In another example embodiment, an apparatus for measuring body impedance is provided that includes means for causing an amplitude modulated signal to be provided to a transmitting electrode that is applied to a body such that the amplitude modulated signal is transmitted through the body, receiving signals, responsive to the amplitude modulated signal, via a receiving electrode that is applied to the body and is remote from the transmitting electrode so as to be in wireless communication with the transmitting electrode via the body, and processing the received signals to determine a measure of the body impedance.

In yet another example embodiment, a computer program product is provided that includes at least one non-transitory computer-readable storage medium having computer-executable program code instructions stored therein. The computer-executable program code instructions include program code instructions configured, upon execution, to cause an amplitude modulated signal to be provided to a transmitting electrode that is applied to a body such that the amplitude modulated signal is transmitted through the body. The program code instructions are also configured to receive signals, responsive to the amplitude modulated signal, via a receiving electrode that is applied to the body and is remote from the transmitting electrode so as to be in wireless communication with the transmitting electrode via the body. The program code instructions are further configured to process the received signals to determine a measure of the body impedance.

### BRIEF DESCRIPTION OF THE DRAWINGS

Having thus described certain example embodiments of the present disclosure in general terms, reference will hereinafter be made to the accompanying drawings, which are not necessarily drawn to scale, and wherein:
Figure 1 is a perspective view of a subject to which a transmitting electrode and a receiving electrode have been applied and are in wireless communication via the body in accordance with an example embodiment of the present disclosure;
Figure 2 is a block diagram of a transmitter of a body impedance measurement apparatus in accordance with an example embodiment of the present disclosure;
Figure 3 is a representation of an amplitude modulated pulse train having a sawtooth shape that is provided by a transmitter of a body impedance measurement apparatus of an example embodiment of the present disclosure;
Figure 4 is a representation of an amplitude modulated square wave having a sawtooth shape that is provided by a transmitter of a body impedance measurement apparatus of an example embodiment of the present disclosure;
Figure 5 is a representation of an amplitude modulated sine wave having a sawtooth shape that is provided by a transmitter of a body impedance measurement apparatus of an example embodiment of the present disclosure;
Figure 6 is a block diagram of a receiver of a body impedance measurement apparatus of an example embodiment of the present disclosure;
Figure 7 is a representation of the amplitude modulated pulses received via the receiving electrode and following any analog front end processing and the rectangular output waveform generated by a receiver based thereupon in accordance with an example embodiment of the present disclosure;
Figure 8 is a representation of the amplitude modulated square wave received via the receiving electrode and following any analog front end processing in accordance with an example embodiment of the present disclosure;
Figure 9 is a representation of the amplitude modulated sine wave received via the receiving electrode and prior to any analog front end processing in accordance with an example embodiment of the present disclosure; and
Figure 10 is a flowchart illustrating the operations performed, such as by the transmitter of Figure 2 and the receiver of Figure 6, in accordance with an example embodiment of the present disclosure.

### DETAILED DESCRIPTION

Some embodiments of the present invention will now be described more fully hereinafter with reference to the accompanying drawings, in which some, but not all, embodiments of the invention are shown. Indeed, various embodiments of the invention may be embodied in many different forms and should not be construed as limited to the embodiments set forth herein; rather, these embodiments are provided so that this disclosure will satisfy applicable legal requirements. Like reference numerals refer to like elements throughout. As used herein, the terms "data," "content," "information," and similar terms may be used interchangeably to refer to data capable of being transmitted, received and/or stored in accordance with embodiments of the present invention.

A body impedance measurement apparatus and method are provided in accordance with certain example embodiments, along with a transmitter and a receiver of the body impedance measurement apparatus. The body impedance measurement apparatus determines a measure of the body impedance which may be utilized for a variety of purposes including diagnosis of the health condition, or monitoring of rehabilitation or training undertaken by a subject. The measure of the body impedance provides information regarding the body composition, such as the different types of tissue that comprise the portion of the body being monitored. In this regard, the measure of body impedance may provide information from which the relative percent composition of various types of tissues, such as fat, muscle or the like, may be determined. The measure of body impedance may also provide the information regarding the diameter of blood vessels in the portion of the body subject to the impedance measurement as well as the information regarding the stress level experienced by the subject and/or the blood supply to the portion of the body subjected to the impedance measurement. Still further, the measure of the body impedance may be utilized in conjunction with impedance plethysmography (IPG) for detecting blood volume changes as may be indicative of venous thrombosis and/or the measurement of electrodermal activity.

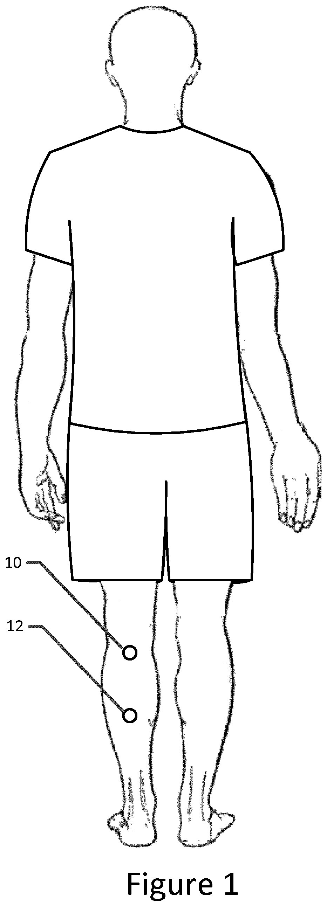

The body impedance measurement apparatus includes transmitter means, such as a transmitter, including transmitting electrode means, such as a transmitting electrode, configured to be applied to the body. Similarly, the body impedance measurement apparatus includes receiver means, such as a receiver, including receiving electrode means, such as a receiving electrode, configured to be applied to the body. The transmitting electrode 10 is applied to the body so as to be remote from, that is, spaced from, the receiving electrode 12 as shown in Figure 1 (for example). The transmitting and receiving electrodes may be applied to different portions of the body in order to measure the body impedance of the respective portions of the body. In the illustrated embodiment, the transmitting and receiving electrodes are applied to the lower leg or calf of the subject. However, the transmitting and receiving electrodes may be applied to other portions of the body including the upper leg, the upper arm, the lower arm, the chest or the like.

The transmitting and receiving electrodes 10, 12 are formed of a conductive material. While the transmitting and receiving electrodes may be formed of various conductive materials, the transmitting and receiving electrodes of an example embodiment are formed of stainless steel. The transmitting and receiving electrodes may be releasably attached to respective portions of the body in various manners, such as by an adhesive, e.g., an electrically conductive adhesive. In one embodiment, the transmitting and/or receiving electrodes are disposable or replaceable electrodes that are comprised of an adhesive pad having a metal contact and a connector configured to interconnect the metal contact to a lead connected to a transmitter or receiver. In order to facilitate the transmission of electrical signals between the body and the transmitting and receiving electrodes, a couplant, such as a conductivity gel, may be applied between the body and the transmitting and receiving electrodes. In one embodiment the couplant is carried by the surface of the transmitting and receiving electrodes that is to be placed into contact with the body.

While a conventional bio-impedance monitor may adequately collect bio-impedance measurements in an instance in which the subject is stationary, such as in an instance in which the subject is in the hospital, in a doctor's office or even in a stationary position at home, bio-impedance monitors are increasingly being worn over an extended period of time while the subject performs his or her daily activities. In instances in which the pair of transmitting electrodes and the pair of receiving electrodes of such a conventional bio-impedance monitor are wirelessly connected, however, the resulting bio-impedance monitor may be cumbersome to be worn by the subject for an extended period of time as a result of the difficulties associated with wearing clothing over the bio-impedance monitor without disconnecting or otherwise adversely affecting the performance of the bio-impedance monitor and further as a result of the impediment, either real or imagined, upon the movement of the subject imposed by the wired connection of the respective pairs of electrodes.

In contrast and as shown in Figure 1, the transmitting and receiving electrodes 10, 12 are not connected by wires, but are, instead, in wireless communication via the body as described below. By permitting the transmitting and receiving electrodes to be in wireless communication, the body impedance measurement apparatus may be more readily worn by a subject while performing their daily activities without impeding those activities. As such, the body impedance measurement apparatus and method facilitate the collection of information from which a measure of the body impedance may be determined over a period of time in which the subject is not in a hospital or doctor's office, but is, instead, performing their daily activities without limitation.

Although the example embodiment depicted in Figure 1 includes a single transmitting electrode 10 and a single receiving electrode 12, the transmitting and receiving electrodes may be differently configured in other embodiments. For example, the transmitting electrode of another embodiment includes two conductive plates spaced apart from one another and releasably attached to different respective portions of the body. In this example embodiment, one plate is utilized to introduce a signal into the body, while the other plate is held at ground or a reference potential. Similarly, the receiving electrode of another embodiment includes two conductive plates spaced apart from one another and releasably attached to different respective portions of the body. In this example embodiment, one plate is utilized to receive signals from the body, while the other plate is held at ground or a reference potential. In yet another embodiment in which both the transmitting and receiving electrodes include two conductive plates, the transmitting and receiving electrodes can each utilize the same conductive plate that is held at ground or a reference potential such that the transmitting and receiving electrodes comprise three conductive plates in total.

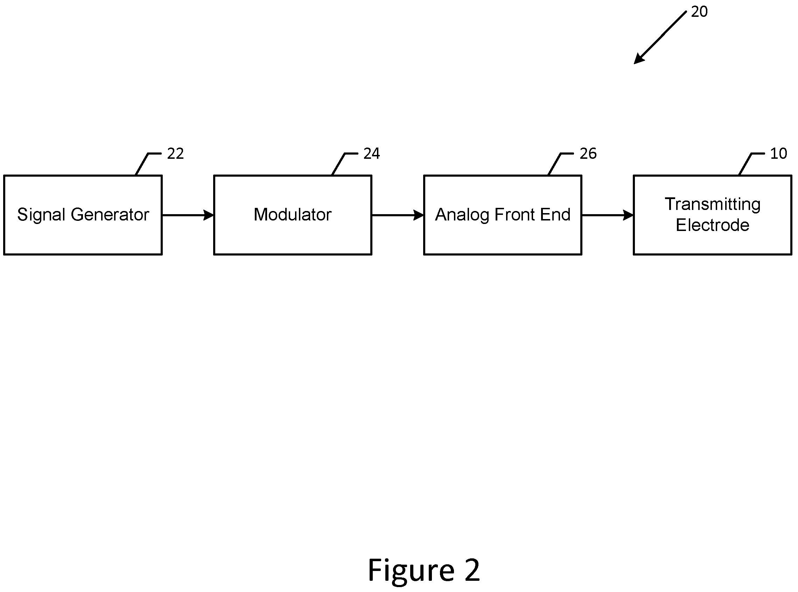

A transmitter means, such as the transmitter, is configured to provide an amplitude modulated signal to the transmitting electrode 10. As shown in Figure 2, the transmitter 20 of an example embodiment includes signal generator means, such as a signal generator 22, configured to generate a first signal. In one embodiment, the first signal comprises a plurality of pulses, such as a plurality of impulses of near-zero duration. The plurality of pulses are generated in accordance with an example embodiment at a predetermined pulse repetition rate. By increasing the frequency at which the pulses are generated, the resolution with which the measure of body impedance is generated may be increased. In one embodiment, the pulses generated by the signal generator have the same amplitude. As discussed below, however, the signal generator may be configured to generate other types of first signals including a first signal having a repeating waveform, such as a square wave or a sine wave, in other embodiments. In these other embodiments, the frequency of the repeating waveform, such as a square wave or a sine wave, is related to the resolution with which body impedance is measured and, as such, the resolution with which the body impedance is measured may be increased by increasing the frequency of the repeating waveform.

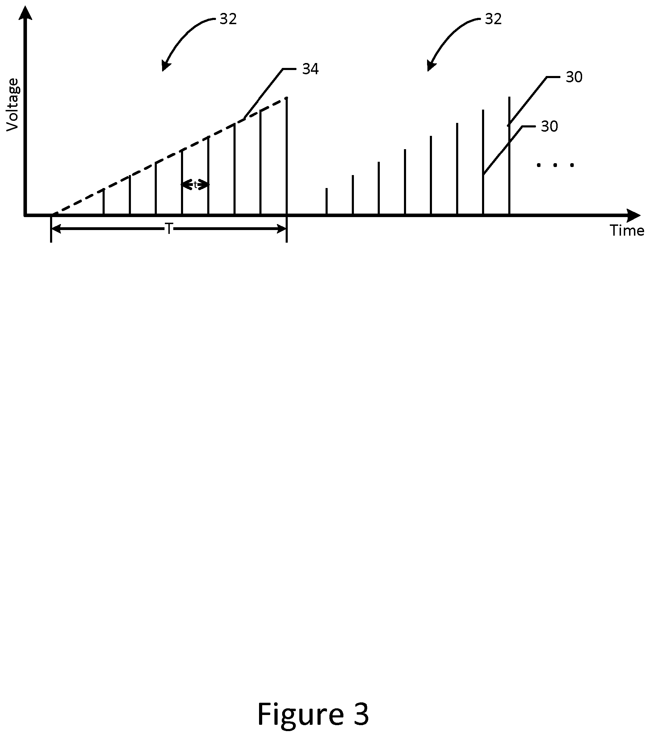

In the embodiment of Figure 2, the transmitter means also includes modulator means, such as a modulator 24, configured to modulate the amplitude of the first signals with an amplitude modulation waveform, such as a sawtooth envelope as described below, to generate an amplitude modulated signal. The amplitude modulation, such as the amplitude modulation waveform, repeats at a predefined frequency. The predetermined pulse repetition rate at which the pulses are generated or the frequency of repeating waveform, such as a square wave or a sine wave, is typically at least twice and, in some embodiments, several orders of magnitude larger than the predefined frequency of the amplitude modulation, such as the amplitude modulation waveform. For example, the predetermined pulse repetition rate at which the pulses are generated is at least 100 times and, in one embodiment, 1,000 times greater than the predefined frequency of the amplitude modulation waveform that defines the frequency at which the amplitude modulated pulse train repeats. As shown in Figure 3, the modulator of an example embodiment is configured to modulate the pulses 30 with a sawtooth envelope 34 to generate an amplitude modulated pulse train 32 having a sawtooth shape that repeats at the predefined frequency. As shown in Figure 3, the sawtooth envelope has a positive, linear slope representative of a linear increase in the amplitude of the modulated pulses throughout the period T. Alternatively, the sawtooth envelope may be a linearly decreasing waveform in which the modulated pulses linearly decrease in amplitude throughout the period T. Still further, the amplitude modulation waveform need not be defined by a sawtooth envelope and need not increase or decrease in a linear fashion, but may have other shapes so long as the amplitude of the modulated pulses continually increases or continually decreases throughout the period T.

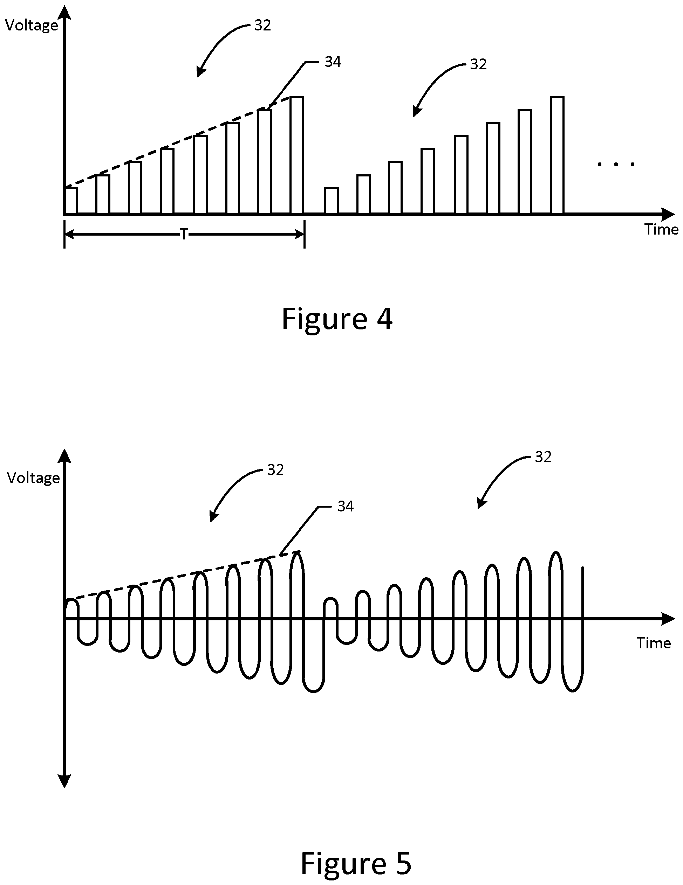

In other embodiments in which the first signal is a repeating waveform, such as a square wave or a sine wave, the repeating waveform may be similarly subjected to amplitude modulation with an amplitude modulation waveform, such as a sawtooth envelope that is repeated at the predefined frequency. For example, Figures 4 and 5 illustrate a square wave and a sine wave, respectively, that are amplitude modulated by the modulator 24 in accordance with a sawtooth envelope 34 so as to have a sawtooth shape that repeats at the predefined frequency.

The rate at which the amplitude of the amplitude modulated signals generated by the modulator 24 increases or decreases and the slope of resulting sawtooth shape 34 of the amplitude modulated signals are defined by the slope of the amplitude modulation waveform, such as a sawtooth envelope. The rate at which the amplitude of the amplitude modulated signals changes as exemplified by the slope of resulting sawtooth shape of the amplitude modulated signals at least partially defines the sensitivity with which the body impedance is measured. In this regard, amplitude modulated signals having an amplitude that increase or decrease at a greater rate such that the resulting sawtooth shape has a greater slope provide for greater sensitivity with respect to the measurement of the body impedance than amplitude modulated signals that increase or decrease at a smaller rate such that the resulting sawtooth shape has a smaller slope. In this regard, the rate at which the amplitude of the amplitude modulated signals change over time has a direct relationship to, such as a direct proportional relationship to, the sensitivity with which the body impedance is measured. In one embodiment, the slope of the sawtooth shape of the amplitude modulated signals is 100 V/ms.

The predefined frequency at which the first signals are amplitude modulated, such as the predefined frequency of the amplitude modulation waveform, such as a sawtooth envelope, may be a predefined frequency within the range of 1 Khz to 50 Mhz. The pulse repetition rate, that is, the frequency at which the pulses are generated, or the frequency of a repeating waveform, such as a sine wave or a square wave, is at least twice as large as the predefined frequency and, in some embodiments at least 100 times or 1000 times as large in order to provide a desired resolution or sensitivity in the body impedance measurements. In some embodiments, the predefined frequency is at the higher end of the above frequency range, such as a predefined frequency being selected from within the range of 10 Mhz to 50 Mhz to improve sensitivity and to reduce the impact of capacitance. By causing the amplitude modulated signals to be amplitude modulated at a predefined frequency at the higher end of the frequency range, the deleterious impact of capacitance may be reduced. In this regard, capacitance, such as capacitance between the transmitting and receiving electrodes 10, 12 and the body the capacitance of the body itself, affects the amplitude modulated signal during the transmission of the amplitude modulated signal between the transmitting and receiving electrodes with the affect caused by capacitance being reduced at higher frequencies such that the signals received by the receiver are more directly related to the body resistance as opposed to the capacitance between the transmitting and receiving electrodes and the body.

The transmitter 20 of the illustrated embodiment also includes an analog front end 26, such as an amplifier, e.g., a radio frequency (RF) amplifier, and optionally a filter configured to reduce the noise present in the amplified signal. In some embodiments, such as in which the amplitude modulated signal is bipolar, such as an amplitude modulated sine wave having positive and negative cycles, the analog front end may also optionally include amplitude limiting circuitry to remove any negative cycles from the amplitude modulated signal. However, the transmitter need not necessarily include amplitude limiting circuitry, as the receiver 40 of at least some embodiments includes amplitude limiting circuitry to remove any negative cycles from the amplitude modulated signal prior to further analysis of the received signal. Thereafter, the amplitude modulated signal is provided to the transmitting electrode 10 for transmission through the body. During propagation through the body, the magnitude of the amplitude modulated waveform is generally reduced as a result of the body impedance such that the signals received by the receiving electrode 12 are indicative of the body impedance.

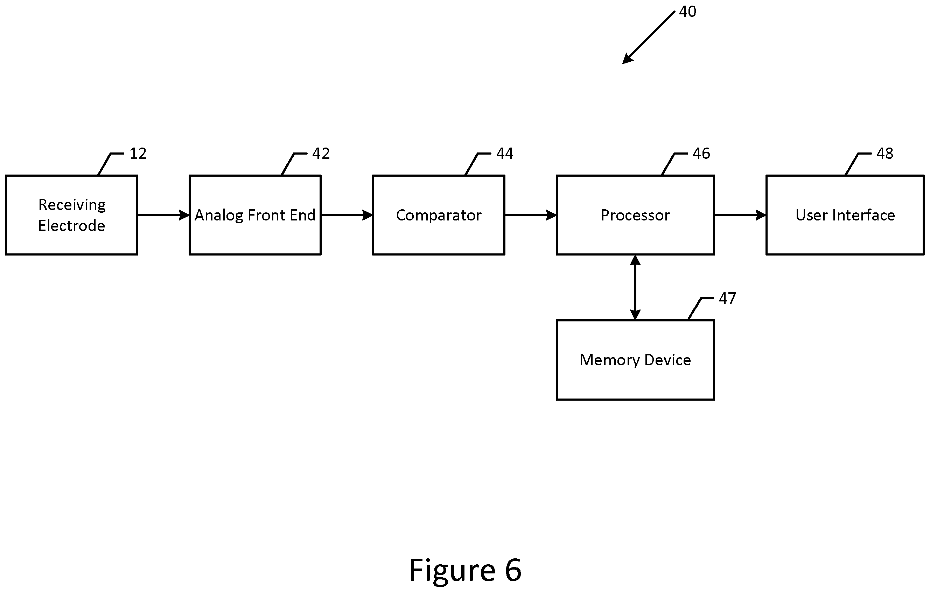

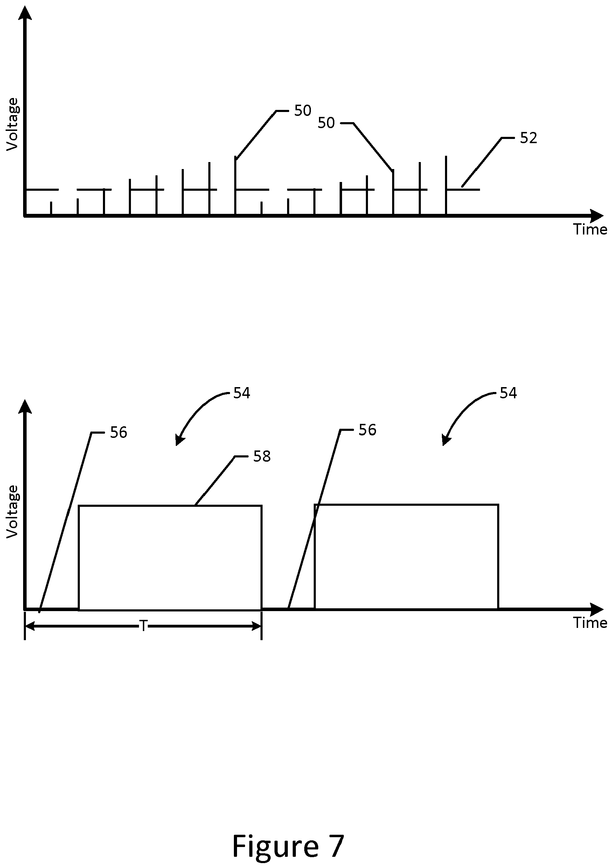

As noted above and as shown in Figure 6, the receiver 40 includes a receiving electrode 12 configured to receive signals responsive to the amplitude modulated signal. In this regard, the receiving electrode is configured to receive the amplitude modulated signal following propagation through the portion of the body between the transmitting electrode 10 and the receiving electrode and following the attenuation of the amplitude modulated signal attributable to the body impedance. As shown in Figure 7, in an example embodiment in which the amplitude modulated signal is an amplitude modulated pulse train, the receiving electrode is configured to receive pulses 50 that form an amplitude modulated pulse train, albeit an amplitude modulated pulse train comprised of pulses having smaller amplitudes than the corresponding pulses of the amplitude modulated pulse train transmitted by the transmitting electrode. However, the amplitude modulated pulse train received by the receiving electrode of the illustrated embodiment is comprised of pulses having the same pulse repetition rate as the pulses transmitted by the transmitting electrode, and still has a sawtooth envelope that repeats at the same predefined frequency as the amplitude modulated pulse train transmitted by the transmitting electrode. As a result of the reduction in magnitude of the pulses received by the receiving electrode relative to the pulses transmitted by the transmitting electrode, the rate of change in amplitude from one pulse to the next pulse as represented by the slope of the sawtooth envelope for the pulses received by the receiving electrode is smaller than that of the sawtooth envelope with which the pulses transmitted by the transmitting electrode were modulated.

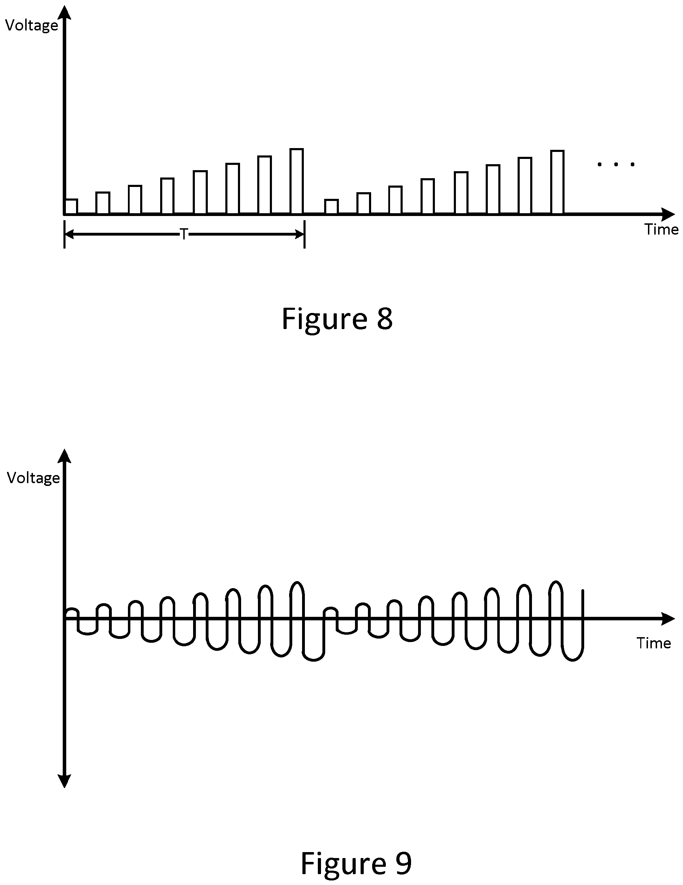

In other embodiments in which the amplitude modulated signal is a repeating waveform, such as a square wave or a sine wave, the receiving electrode 12 is configured to receive the repeating waveform transmitted by the transmitting electrode 10, albeit a repeating waveform, such as a square wave as shown in Figure 8 or a sine wave as shown in Figure 9, that has smaller peak amplitudes than the corresponding peaks of the amplitude modulated repeating waveform transmitted by the transmitting electrode. However, the amplitude modulated repeating waveform received by the receiving electrode of the illustrated embodiment has the same frequency as the frequency of the repeating waveform transmitted by the transmitting electrode, and is amplitude modulated with a sawtooth envelope that repeats at the same predefined frequency as the amplitude modulated signal transmitted by the transmitting electrode. As a result of the reduction in magnitude of the repeating waveform received by the receiving electrode relative to the repeating waveform transmitted by the transmitting electrode, the rate of change in amplitude from the peak amplitude of one cycle to the peak amplitude of the next cycle as represented by the slope of the sawtooth envelope for the repeating waveform, such as a square wave or a sine wave, received by the receiving electrode is smaller than that of the sawtooth envelope with which the repeating waveform transmitted by the transmitting electrode was modulated.

The receiver 40 of an example embodiment also includes an analog front end 42, such as an amplifier, e.g., an RF amplifier, in order to increase the amplitude of the signals received by the receiving electrode 12 and optionally a filter to filter out or remove noise from the signals. In one embodiment, the analog front end also optionally includes amplitude limiting circuitry to remove any negative signals, such as negative cycles from a repeating waveform, such as the negative cycles of the sine wave of Figure 9 or negative signals introduced during propagation through the body, from the received signals. The receiver of an example embodiment also includes comparator means, such as a comparator 44, e.g., comparator circuitry configured to perform a comparison. The comparator is configured to compare the received signals to a threshold level 52. In this regard, the comparator is configured to compare the amplitude of the amplitude modulated signals received by the receiving electrode to the threshold level and to classify the signals as either being less than or greater than the threshold level. Although the threshold level may be established at any of various different levels, the threshold level is generally established at an intermediate value relative to the amplitudes of the amplitude modulated signals received by the receiving electrode such that some of the signals have a greater amplitude than the predefined threshold and some of the signals have a smaller amplitude than the predefined threshold level.

As a result of the comparison of the received signals to the threshold level, the comparator 44 of an example embodiment is also configured to generate a rectangular output waveform having the same predefined frequency as the predefined frequency of the amplitude modulation waveform, such as a sawtooth envelope, with which the first signals are modulated prior to being transmitted by the transmitting electrode 10. In relation to the generation of a rectangular output waveform from an amplitude modulated pulse train received by the receiver 40, Figure 7 depicts two cycles of the resulting rectangular output waveform. Similar rectangular output waveforms may be generated from amplitude modulated repeating waveforms, such as a square wave or a sine wave, received by the receiver in other embodiments. The rectangular output waveform alternates between a smaller amplitude representative of pulses having an amplitude less than the threshold level and a larger amplitude representative of pulses having an amplitude greater than the threshold level. In an embodiment in which the pulses consistently increase in amplitude throughout the period T as shown in Figure 7 or consistently decrease in amplitude throughout the period T, the rectangular output pulse has only two portions, namely, a first portion 56 having the smaller amplitude and a second portion 58 having the larger amplitude. The rectangular output waveform generated by the comparator has a duty cycle that defines the percent of the period T of the rectangular output waveform having a predefined one of the two different amplitudes, such as the percentage of the period for which the rectangular output waveform has the higher amplitude, as opposed to the smaller amplitude.

The duty cycle of the rectangular output waveform provides the measure of the body impedance. As used herein, reference to the body impedance relates to the absolute value of the body impedance without consideration of the phase angle. In this regard, a rectangular output waveform having a larger duty cycle attributable to a greater percentage of the rectangular output waveform having the higher amplitude is representative of a lower body impedance than a rectangular output waveform having a smaller duty cycle. In this regard, a larger duty cycle is indicative of less attenuation of the amplitude modulated signals during propagation through the portion of the body between the transmitting and receiving electrodes 10, 12 and, as a result, less body impedance. As a result of less body impedance and correspondingly less attenuation, more of the signals received by the receiving electrode have an amplitude greater than the threshold level 52 which, in turn, results in a greater percentage of the rectangular output waveform having the higher amplitude. Conversely, a rectangular output waveform having a smaller duty cycle attributable to a smaller percentage of the rectangular output waveform having the higher amplitude is representative of a larger body impedance than a rectangular output waveform having a larger duty cycle. In this regard, a smaller duty cycle is indicative of more attenuation of the amplitude modulated signals during propagation through the portion of the body between the transmitting and receiving electrodes and, as a result, more body impedance. As a result of more body impedance and correspondingly more attenuation, fewer of the signals received by the receiving electrode have an amplitude greater than the threshold level which, in turn, results in a smaller percentage of the rectangular output waveform having the higher amplitude. As illustrated by the foregoing examples, the duty cycle of the rectangular output waveform may have an inverse relationship, such as an inverse proportional relationship, to the body impedance of the portion of the body between the transmitting and receiving electrodes. However, an inverse relationship is just one example of a plurality of relationships that may be defined between the duty cycle of the rectangular output waveform and the body impedance of the portion of the body between the transmitting and receiving electrodes and other relationships, such as a direct proportional relationship, may be defined in other example embodiments.

As shown in Figure 6, the receiver 40 of an example embodiment also includes a processor 46 configured to generate the measure of the body impedance based upon the duty cycle. The processor may be embodied in a number of different ways. For example, the processor may be embodied as one or more of various hardware processing means such as a coprocessor, a microprocessor, a controller, a digital signal processor (DSP), a processing element with or without an accompanying DSP, or various other circuitry including integrated circuits such as, for example, an ASIC (application specific integrated circuit), an FPGA (field programmable gate array), a microcontroller unit (MCU), a hardware accelerator, a special-purpose computer chip, or the like.

As such, in some embodiments, the processor may include one or more processing cores configured to perform independently. A multi-core processor may enable multiprocessing within a single physical package. Additionally or alternatively, the processor may include one or more processors configured in tandem via the bus to enable independent execution of instructions, pipelining and/or multithreading.

In an example embodiment, the processor 46 may be configured to execute instructions stored in a memory device 47 or otherwise accessible to the processor. Alternatively or additionally, the processor may be configured to execute hard coded functionality. As such, whether configured by hardware or software methods, or by a combination thereof, the processor may represent an entity (e.g., physically embodied in circuitry) capable of performing operations according to an embodiment of the present disclosure while configured accordingly. Thus, for example, when the processor is embodied as an ASIC, FPGA or the like, the processor may be specifically configured hardware for conducting the operations described herein. Alternatively, as another example, when the processor is embodied as an executor of instructions, the instructions may specifically configure the processor to perform the algorithms and/or operations described herein when the instructions are executed. The processor may include, among other things, a clock, an arithmetic logic unit (ALU) and logic gates configured to support operation of the processor.

In an embodiment in which the body impedance measuring apparatus is worn and obtains measurements over a period of time, such as during rehabilitation, training or the like, the processor 46 may provide a relative measure of the body impedance with respect to prior measures of body impedance, such as an indication that the body impedance is increasing or decreasing. Alternatively, the processor may be configured to convert the duty cycle to a measure of the body impedance. For a predefined amplitude modulated signal transmitted by the transmitter 20, the processor may be configured to convert the duty cycle to a respective measure of the body impedance, such as based upon a predefined relationship between the duty cycle and the body impedance, such as may be stored in a memory device 47 accessible by the processor. This relationship between the duty cycle and the body impedance may be predefined based upon measurements of the duty cycle of a rectangular output waveform for subjects having different, known body impedance. In this regard, the memory device may store a lookup table that correlates the duty cycle of the rectangular output waveform to a measure of the body impedance. The measure of the body impedance determined by the processor may be stored, such as by the memory device for subsequent analysis by a physician or other healthcare professional. Based upon the analysis, the physician or other healthcare professional may determine the body composition of the subject, such as the relative compositional percentages of different types of tissue and/or one or more other parameters, such as the diameter of one or more blood vessels, the stress level experienced by the subject, the blood supply throughout the portion of the body between the transmitting and receiving electrodes 10, 12, the electrodermal activity of the subject and/or the blood volume changes per IPG as may be indicative of venous thrombosis.

As the body impedance measurement apparatus is configured to be worn in some embodiments for an extended period of time, the receiver 40 of an example embodiment also includes or is associated with a user interface 48, such as a display screen, for providing information regarding the measure of the body impedance to the subject, a physician or the healthcare professional or the like. Although the receiver may include a user interface, such as a display integral with the other components of the receiver, the receiver of an example embodiment provides the user interface in the form of the user interface of another device carried by or worn by the subject, such as a smartwatch, a smartphone or the like. In this regard, the processor 46 of the receiver is configured to cause signals representative of the body impedance that have been generated by the processor to be transmitted to the user interface, such as wirelessly to the user interface of a smartwatch, a smartphone or the like, for presentation of a representation of the measure of the body impedance.

In the foregoing embodiments, the signal generator 22, the modulator 24 and the comparator 44 may be comprised of various means including, for example, signal generation circuitry, e.g. a pulse generator, a square wave generator or a sine wave generator, modulator circuitry and comparator circuitry, respectively. As used herein, the term 'circuitry' refers to (a) hardware-only circuit implementations (e.g., implementations in analog circuitry and/or digital circuitry); (b) combinations of circuits and computer program product(s) comprising software and/or firmware instructions stored on one or more computer readable memories that work together to cause an apparatus to perform one or more functions described herein; and (c) circuits, such as, for example, a microprocessor(s) or a portion of a microprocessor(s), that require software or firmware for operation even if the software or firmware is not physically present. This definition of 'circuitry' applies to all uses of this term herein, including in any claims. As a further example, as used herein, the term 'circuitry' also includes an implementation comprising one or more processors and/or portion(s) thereof and accompanying software and/or firmware. As another example, the term 'circuitry' as used herein also includes, for example, an integrated circuit in a field programmable gate array, and/or other computing device.

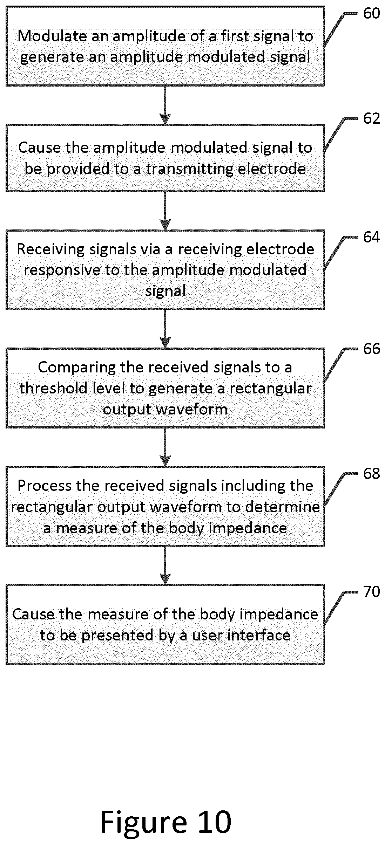

The operations for body impedance measurement that are performed in accordance with an example embodiment are depicted in Figure 10. As shown in block 60, after having generated a first signal, such as by a signal generator 22, the amplitude of the first signal is modulated, such as by the modulator 24, in order to generate an amplitude modulated signal that repeats at a predefined frequency. The amplitude modulated signal is subjected to analog processing, such as by analog front end 26 in one embodied, prior to being caused to be provided to a transmitting electrode 10 that has been applied to the body. See block 62 of Figure 10. As such, the amplitude modulated signal is transmitted through the body.

Following transmission through the body, signals are received, such as by a receiving electrode 12 that has also been applied to the body, with the received signals being responsive to the amplitude modulated signal. See block 64. As shown in Figure 1 and as described above, the receiving electrode is remote from the transmitting electrode 10 and is in wireless communication with the transmitting electrode via the body. After analog processing by the analog front end 42, the received signals are compared, such as with a comparator 44, to a threshold level. See block 66. In one embodiment, the comparison of the received signals to the threshold level generates a rectangular output waveform. The received signals and, more typically, the rectangular output waveform generated from the received signals are then processed, such as by the processor 46, in order to determine a measure of the body impedance, such as the absolute value of the body impedance. See block 68 of Figure 10. As described above, the measure of the body impedance may be based upon the duty cycle of the rectangular output waveform. In one embodiment, the measure of the body impedance is then caused to be presented by user interface 48 for review, for example, by the subject. See block 70. In one example, the user interface is provided by another device carried or worn by the subject, such as a smartwatch, a smartphone or the like, thereby facilitating provision of information regarding the body impedance to the subject wearing the body impedance measurement apparatus.

As described above, a body impedance measurement apparatus and method as well as the transmitter 20 and receiver 40 of a body impedance measurement apparatus are provided in accordance with an example embodiment. The body impedance measurement apparatus and method permit information from which a measure of the body impedance to be determined while relying upon wireless communication between the transmitting and receiving electrodes 10, 12, even though the remainder of the transmitter and the receiver may be connected, such as via respective wired connections, to the transmitting electrode and the receiving electrode, respectively. As such, the body impedance measurement apparatus and method may be integrated into a body worn system without inhibiting performance of the subject's daily activities.

As described above, Figure 8 is a flowchart of an apparatus, method, and computer program product according to example embodiments of the invention. It will be understood that each block of the flowchart, and combinations of blocks in the flowchart, may be implemented by various means, such as hardware, firmware, processor, circuitry, and/or other devices associated with execution of software including one or more computer program instructions. For example, one or more of the procedures described above may be embodied by computer program instructions that causes signals to be transmitted to and received from the electrodes and that subsequently processes the signals received by the receiver 40. In this regard, the computer program instructions which embody the procedures described above may be stored by a memory device of an apparatus employing an embodiment of the present invention and executed by a processor of the apparatus. As will be appreciated, any such computer program instructions may be loaded onto a computer or other programmable apparatus (e.g., hardware) to produce a machine, such that the resulting computer or other programmable apparatus implements the functions specified in the flowchart blocks. These computer program instructions may also be stored in a computer-readable memory that may direct a computer or other programmable apparatus to function in a particular manner, such that the instructions stored in the computer-readable memory produce an article of manufacture, the execution of which implements the function specified in the flowchart blocks. The computer program instructions may also be loaded onto a computer or other programmable apparatus to cause a series of operations to be performed on the computer or other programmable apparatus to produce a computer-implemented process such that the instructions which execute on the computer or other programmable apparatus provide operations for implementing the functions specified in the flowchart blocks.

A computer program product is therefore defined in those instances in which the computer program instructions, such as computer-readable program code portions, are stored by at least one non-transitory computer-readable storage medium with the computer program instructions, such as the computer-readable program code portions, being configured, upon execution, to perform the functions described above, such as in conjunction with the flowchart of Figure 8. In other embodiments, the computer program instructions, such as the computer-readable program code portions, need not be stored or otherwise embodied by a non-transitory computer-readable storage medium, but may, instead, be embodied by a transitory medium with the computer program instructions, such as the computer-readable program code portions, still being configured, upon execution, to perform the functions described above.

Accordingly, blocks of the flowchart support combinations of means for performing the specified functions and combinations of operations for performing the specified functions for performing the specified functions. It will also be understood that one or more blocks of the flowchart, and combinations of blocks in the flowchart, may be implemented by special purpose hardware-based computer systems which perform the specified functions, or combinations of special purpose hardware and computer instructions.

In some embodiments, certain ones of the operations above may be modified or further amplified. Furthermore, in some embodiments, additional optional operations may be included. Modifications, additions, or amplifications to the operations above may be performed in any order and in any combination.

Many modifications and other embodiments of the inventions set forth herein will come to mind to one skilled in the art to which these inventions pertain having the benefit of the teachings presented in the foregoing descriptions and the associated drawings. Therefore, it is to be understood that the inventions are not to be limited to the specific embodiments disclosed and that modifications and other embodiments are intended to be included within the scope of the appended claims. Moreover, although the foregoing descriptions and the associated drawings describe example embodiments in the context of certain example combinations of elements and/or functions, it should be appreciated that different combinations of elements and/or functions may be provided by alternative embodiments without departing from the scope of the appended claims. In this regard, for example, different combinations of elements and/or functions than those explicitly described above are also contemplated as may be set forth in some of the appended claims. Although specific terms are employed herein, they are used in a generic and descriptive sense only and not for purposes of limitation.

## Claims

1. An apparatus comprising:
transmitter means (20) for providing an amplitude modulated signal via a transmitting electrode means (10) to a body to which the transmitting electrode means is configured to be applied, wherein the transmitter means further comprises modulator means (24) configured to modulate an amplitude of a first signal, being a plurality of pulses, in accordance with an amplitude modulation waveform to generate an amplitude modulated signal for which amplitude modulation is repeated at a predefined frequency, the amplitude modulated signal being in the form of an amplitude modulated pulse train; and
receiver means (40) for receiving signals, responsive to the amplitude modulated signal, via a receiving electrode means (12) that is configured to be applied to the body and to be remote from the transmitting electrode means so as to be in wireless communication with the transmitting electrode means via the body, the receiver means being configured to process the received signals to determine a measure of a body impedance; wherein the receiver means comprises:
comparator means configured to compare the received signals to a threshold level in order to generate a rectangular output waveform having the predefined frequency, wherein the rectangular output waveform has a duty cycle that is indicative of the measure of the body impedance; and
processor means configured to determine the measure of the body impedance based on upon a predefined relationship between duty cycle and body impedance, wherein the predefined relationship is stored in a lookup table of a memory device and is identified based on correlating duty cycles of rectangular output waveforms to known measures of body impedance for multiple subjects.

2. An apparatus according to claim 1, wherein the first signal is amplitude modulated using an amplitude modulation waveform with a predetermined frequency within the range of 1 Khz to 50 Mhz.

3. An apparatus according to Claim 2 wherein the amplitude modulation waveform comprises a sawtooth envelope, and wherein the modulator means is configured to modulate the amplitude of the first signal in accordance with the sawtooth envelope to generate the amplitude modulated signal having a sawtooth shape that repeats at the predefined frequency.

4. A method comprising:
modulating an amplitude of a first signal, the first signal comprising a plurality of pulses, in accordance with an amplitude modulation waveform, to generate an amplitude modulated signal for which amplitude modulation is repeated at a predefined frequency, the amplitude modulated signal being in the form of an amplitude modulated pulse train;
causing the amplitude modulated signal to be provided to a transmitting electrode that is applied to a body such that the amplitude modulated signal is transmitted through the body;
receiving signals, responsive to the amplitude modulated signal, via a receiving electrode that is applied to the body and is remote from the transmitting electrode so as to be in wireless communication with the transmitting electrode via the body; and
comparing the received signals to a threshold level in order to generate a rectangular output waveform having the predefined frequency, wherein the rectangular output waveform also has a duty cycle that is indicative of the measure of the body impedance; processing the received signals to determine a measure of a body impedance based on upon a predefined relationship between duty cycle and body impedance, wherein the predefined relationship is stored in a lookup table of a memory device and identified based on correlating duty cycles of rectangular output waveforms to known measures of body impedance for multiple subjects.

5. A method according to claim 4 wherein the first signal is amplitude modulated using an amplitude modulated waveform with a predefined frequency within the range of 1Khz to 50Mhz.

6. A method according to Claim 5 wherein the amplitude modulation waveform comprises a sawtooth envelope, and wherein modulating the amplitude of the first signal comprises modulating the amplitude of the first signal in accordance with the sawtooth envelope to generate the amplitude modulated signal having a sawtooth shape that repeats at the predefined frequency.

7. A computer program product comprising at least one non-transitory computer-readable storage medium having computer-executable program code instruction stored therein, the computer-executable program code instructions comprising program code instructions configured, upon execution, to perform the method according to any one of Claims 4 to 6.

## Patentansprüche

1. Einrichtung, die Folgendes umfasst:
ein Sendermittel (20) zum Bereitstellen eines amplitudenmodulierten Signals via ein Sendeelektrodenmittel (10) für einen Körper, an den das Sendeelektrodenmittel ausgelegt ist, angelegt zu werden, wobei das Sendermittel ferner ein Modulatormittel (24) umfasst, das dazu ausgelegt ist, eine Amplitude eines ersten Signals, bei dem es sich um eine Vielzahl von Impulsen handelt, gemäß einer Amplitudenmodulationswellenform zu modulieren, um ein amplitudenmoduliertes Signal zu erzeugen, für das eine Amplitudenmodulation mit einer vordefinierten Frequenz wiederholt wird, wobei das amplitudenmodulierte Signal die Form einer amplitudenmodulierten Impulsfolge aufweist; und
ein Empfängermittel (40) zum Empfangen von Signalen in Reaktion auf das amplitudenmodulierte Signal via ein Empfangselektrodenmittel (12), das dazu ausgelegt ist, an den Körper angelegt zu werden und vom Sendeelektrodenmittel entfernt zu sein, um mit dem Sendeelektrodenmittel via den Körper in drahtloser Kommunikation zu stehen, wobei das Empfängermittel dazu ausgelegt ist, die empfangenen Signale zu verarbeiten, um ein Maß einer Körperimpedanz zu bestimmen; wobei das Empfängermittel Folgendes umfasst:
ein Komparatormittel, das dazu ausgelegt ist, die empfangenen Signale mit einem Schwellwertpegel zu vergleichen, um eine Rechteckausgangswellenform zu erzeugen, die die vordefinierte Frequenz aufweist, wobei die Rechteckausgangswellenform ein Tastverhältnis aufweist, dass das Maß der Körperimpedanz anzeigt; und
ein Prozessormittel, das dazu ausgelegt ist, das Maß der Körperimpedanz auf Basis einer vordefinierten Beziehung zwischen Tastverhältnis und Körperimpedanz zu bestimmen, wobei die vordefinierte Beziehung in einer Nachschlagetabelle einer Speichervorrichtung gespeichert ist und auf Basis des Korrelierens von Tastverhältnissen von Rechteckausgangswellenformen mit bekannten Maßen einer Körperimpedanz für mehrere Testpersonen identifiziert wird.

2. Einrichtung nach Anspruch 1, wobei das erste Signal unter Verwendung einer Amplitudenmodulationswellenform mit einer vorbestimmten Frequenz im Bereich von 1 kHz bis 50 MHz amplitudenmoduliert wird.

3. Einrichtung nach Anspruch 2, wobei die Amplitudenmodulationswellenform eine Sägezahnhüllkurve umfasst, und wobei das Modulatormittel dazu ausgelegt ist, die Amplitude des ersten Signals gemäß der Sägezahnhüllkurve zu modulieren, um das amplitudenmodulierte Signal mit einer Sägezahnform zu erzeugen, das mit der vordefinierten Frequenz wiederholt wird.

4. Verfahren, das Folgendes umfasst:
Modulieren einer Amplitude eines ersten Signals, wobei das erste Signal eine Vielzahl von Impulsen umfasst, gemäß einer Amplitudenmodulationswellenform, um ein amplitudenmoduliertes Signal zu erzeugen, für das eine Amplitudenmodulation mit einer vordefinierten Frequenz wiederholt wird, wobei das amplitudenmodulierte Signal die Form einer amplitudenmodulierten Impulsfolge aufweist;
Bewirken, dass das amplitudenmodulierte Signal einer Sendeelektrode bereitgestellt wird, die an einen Körper angelegt wird, derart, dass das amplitudenmodulierte Signal durch den Körper übertragen wird;
Empfangen von Signalen in Reaktion auf das amplitudenmodulierte Signal via eine Empfangselektrode, die an den Körper angelegt wird und von der Sendeelektrode entfernt ist, um mit der Sendeelektrode via den Körper in drahtloser Kommunikation zu stehen; und
Vergleichen der empfangenen Signale mit einem Schwellwertpegel, um eine Rechteckausgangswellenform zu erzeugen, die die vordefinierte Frequenz aufweist, wobei die Rechteckausgangswellenform auch ein Tastverhältnis aufweist, das das Maß der Körperimpedanz anzeigt; Verarbeiten der empfangenen Signale, um auf Basis einer vordefinierten Beziehung zwischen Tastverhältnis und Körperimpedanz ein Maß der Körperimpedanz zu bestimmen, wobei die vordefinierte Beziehung in einer Nachschlagetabelle einer Speichervorrichtung gespeichert ist und auf Basis des Korrelierens von Tastverhältnissen von Rechteckausgangswellenformwn mit bekannten Maßen einer Körperimpedanz für mehrere Testpersonen identifiziert wird.

5. Verfahren nach Anspruch 4, wobei das erste Signal unter Verwendung einer amplitudenmodulierten Wellenform mit einer vordefinierten Frequenz im Bereich von 1 kHz bis 50 MHz amplitudenmoduliert wird.

6. Verfahren nach Anspruch 5, wobei die Amplitudenmodulationswellenform eine Sägezahnhüllkurve umfasst, und wobei das Modulieren der Amplitude des ersten Signals das Modulieren der Amplitude des ersten Signals gemäß der Sägezahnhüllkurve umfasst, um das amplitudenmodulierte Signal mit einer Sägezahnform zu erzeugen, das mit der vordefinierten Frequenz wiederholt wird.

7. Computerprogrammprodukt, das mindestens ein nichttransitorisches computerlesbares Speichermedium umfasst, in dem computerausführbare Programmcodeanweisungen gespeichert sind, wobei die computerausführbaren Programmcodeanweisungen Programmcodeanweisungen umfassen, die dazu ausgelegt sind, nach Ausführung das Verfahren nach einem der Ansprüche 4 bis 6 durchzuführen.

## Revendications

1. Appareil comprenant :
un dispositif émetteur (20) pour appliquer un signal modulé en amplitude via un dispositif d'électrode de transmission (10) à un corps sur lequel le dispositif d'électrode de transmission est configuré pour être apposé, dans lequel le dispositif émetteur comprend en outre un dispositif modulateur (24) configuré pour moduler une amplitude d'un premier signal qui est une pluralité d'impulsions conformément à une forme d'onde de modulation d'amplitude, pour générer un signal modulé en amplitude pour lequel une modulation d'amplitude est répétée à une fréquence prédéfinie, le signal modulé en amplitude étant sous la forme d'un train d'impulsions modulé en amplitude ; et
un dispositif récepteur (40) pour recevoir des signaux en réponse au signal modulé en amplitude via un dispositif d'électrode de réception (12) qui est configuré pour être apposé sur le corps et pour être à distance du dispositif d'électrode de transmission de manière à être en communication sans fil avec le dispositif d'électrode de transmission via le corps, le dispositif récepteur étant configuré pour traiter les signaux reçus pour déterminer une mesure d'une impédance corporelle ; dans lequel le dispositif récepteur comprend :
un dispositif comparateur configuré pour comparer les signaux reçus à un niveau seuil afin de générer une forme d'onde de sortie rectangulaire à la fréquence prédéfinie, dans lequel la forme d'onde de sortie rectangulaire a un rapport cyclique qui est indicatif de la mesure de l'impédance corporelle ; et
un dispositif processeur configuré pour déterminer la mesure de l'impédance corporelle sur la base d'une relation prédéfinie entre rapport cyclique et l'impédance corporelle, dans lequel la relation prédéfinie est stockée dans une table de consultation d'un dispositif de mémoire et est identifiée sur la base d'une corrélation des rapports cycliques des formes d'onde de sortie rectangulaires sur les mesures connues de l'impédance corporelle pour de multiples sujets.

2. Appareil selon la revendication 1, dans lequel le premier signal est modulé en amplitude à l'aide d'une forme d'onde de modulation d'amplitude à une fréquence prédéterminée dans la plage de 1 Khz à 50 Mhz.

3. Appareil selon la revendication 2, dans lequel la forme d'onde de modulation d'amplitude comprend une enveloppe en dents de scie, et dans lequel le dispositif modulateur est configuré pour moduler l'amplitude du premier signal conformément à l'enveloppe en dents de scie pour générer le signal modulé en amplitude ayant une forme en dents de scie qui se répète à la fréquence prédéfinie.

4. Procédé comprenant :
la modulation d'une amplitude d'un premier signal, le premier signal comprenant une pluralité d'impulsions conformément à une forme d'onde de modulation d'amplitude, pour générer un signal modulé en amplitude pour lequel une modulation d'amplitude est répétée à une fréquence prédéfinie,
le signal modulé en amplitude étant sous la forme d'un train d'impulsions modulé en amplitude ;
la provocation de l'application du signal modulé en amplitude à une électrode de transmission qui est apposée sur un corps, de sorte que le signal modulé en amplitude soit transmis à travers le corps ;
la réception de signaux en réponse au signal modulé en amplitude, via une électrode de réception qui est apposée sur le corps et qui est à distance de l'électrode de transmission de manière à être en communication sans fil avec l'électrode de transmission via le corps ; et
la comparaison des signaux reçus à un niveau seuil afin de générer une forme d'onde de sortie rectangulaire à la fréquence prédéfinie, dans lequel la forme d'onde de sortie rectangulaire a également un rapport cyclique qui est indicatif de la mesure de l'impédance corporelle ;
le traitement des signaux reçus pour déterminer une mesure d'une impédance corporelle sur la base d'une relation prédéfinie entre le rapport cyclique et l'impédance corporelle, dans lequel la relation prédéfinie est stockée dans une table de consultation d'un dispositif de mémoire et identifiée sur la base de la corrélation des rapports cycliques des formes d'onde de sortie rectangulaires sur les mesures connues de l'impédance corporelle pour de multiples sujets.

5. Procédé selon la revendication 4 dans lequel le premier signal est modulé en amplitude à l'aide d'une forme d'onde modulée en amplitude à une fréquence prédéfinie dans la plage de 1 Khz à 50 Mhz.

6. Procédé selon la revendication 5, dans lequel la forme d'onde de modulation d'amplitude comprend une enveloppe en dents de scie, et dans lequel la modulation de l'amplitude du premier signal comprend la modulation de l'amplitude du premier signal conformément à l'enveloppe en dents de scie pour générer le signal modulé en amplitude ayant une forme en dents de scie qui se répète à la fréquence prédéfinie.

7. Produit de programme informatique comprenant au moins un support de stockage non transitoire lisible par ordinateur sur lequel sont stockées des instructions de code de programme exécutables par ordinateur, les instructions de code de programme exécutables par ordinateur comprenant des instructions de code de programme configurées, lors de l'exécution, pour réaliser le procédé selon l'une quelconque des revendications 4 à 6.
